(19) Europäisches Patentamt
European Patent Office
Office européen des brevets

(11) Veröffentlichungsnummer : **0 362 668 B1**

(12) # EUROPÄISCHE PATENTSCHRIFT

(45) Veröffentlichungstag der Patentschrift :
**27.01.93 Patentblatt 93/04**

(51) Int. Cl.$^5$ : **C07D 207/28**

(21) Anmeldenummer : **89117716.4**

(22) Anmeldetag : **26.09.89**

(54) **Derivate der 2-Pyrrolidon-5-carbonsäure und Verfahren zu ihrer Gewinnung.**

(30) Priorität : **06.10.88 DE 3833972**

(43) Veröffentlichungstag der Anmeldung :
**11.04.90 Patentblatt 90/15**

(45) Bekanntmachung des Hinweises auf die Patenterteilung :
**27.01.93 Patentblatt 93/04**

(84) Benannte Vertragsstaaten :
**AT BE CH DE ES FR GB GR IT LI LU NL SE**

(56) Entgegenhaltungen :
**DE-C- 3 735 263**
**DE-C- 3 735 264**

(73) Patentinhaber : **Degussa Aktiengesellschaft**
**Weissfrauenstrasse 9**
**W-6000 Frankfurt am Main 1 (DE)**

(72) Erfinder : **Krimmer, Hans-Peter, Dr.**
**Am weissen Turm 48**
**W-6000 Frankfurt am Main 60 (DE)**
Erfinder : **Drauz, Karlheinz, Dr.**
**Zur Marienruhe 13**
**W-6463 Freigericht 1 (DE)**

EP 0 362 668 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft die beiden Enantiomeren des reinen, kristallinen Trihydrats des Natrium-2-pyrrolidon-5-carboxylats der Formel

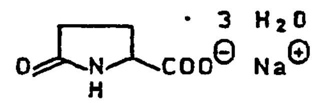

und ein Verfahren zu deren Gewinnung.

Natrium-2-pyrrolidon-5-carboxylat, auch kurz Natriumpyroglutamat genannt, ist zwar in der L- und D-Form und auch als Racemat bekannt. Die kristallinen Trihydrate von Natrium-L- und -D-pyroglutamat sind jedoch neue Verbindungen, die bisher in der Literatur nicht beschrieben wurden.

Diese Verbindungen haben sich als geeignet zur Herstellung von Pestiziden, Arzneimitteln, Kosmetika und insbesondere feuchtigkeitshaltenden Lösungen, Mischungen, Emulsionen, Suspensionen und dergleichen erwiesen.

Gegenstand der Erfindung sind daher die neuen Trihydrate des Natrium-L- und -D-2-pyrrolidon-5-carboxylats.

Ein weiterer Gegenstand der Erfindung ist ein Verfahren zu deren Gewinnung, welches dadurch gekennzeichnet ist, daß man in einer wäßrigen Lösung von Natrium-L- oder D-2-pyrrolidon-5-carboxylat, die höchstens 15 Molprozent des jeweiligen anderen Enantiomeren enthalt, die Konzentration in dem Bereich zwischen 40 und 73,65 Gewichtsprozent und die Temperatur in dem Bereich zwischen -20 °C und +42 °C so aufeinander abstimmt, daß die Löslichkeit des jeweiligen Trihydrats überschritten wird.

Da die Löslichkeit sowohl von der Konzentration als auch von der Temperatur abhängt, kann die Kristallisation einerseits dadurch erzielt werden, daß man aus einer verdünnten wäßrigen Lösung einen Teil des Wassers entfernt und somit eine Aufkonzentration erreicht. Die Entfernung des Wassers kann durch Erhöhung der Temperatur der Lösung oder durch Anlegen von Vakuum oder, am besten, durch eine Kombination dieser beiden Maßnahmen bewerkstelligt werden.

Andererseits gelingt es auch, die Kristallisation dadurch herbeizuführen, daß man die Temperatur der Lösung erniedrigt.

Da die Löslichkeit des jeweiligen Trihydrats mit fallender Temperatur sinkt, ist für die Kristallisation bei jeder Temperatur eine Mindestkonzentration an Natrium-L- oder -D-pyroglutamat erforderlich. Im folgenden bedeuten die Konzentrationsangaben jeweils Gewichtsprozente.

So kristallisieren bei 20 °C aus einer 55 %-igen wäßrigen Lösung von Natrium-L-pyroglutamat etwa 20 % der Gesamtmenge als Trihydrat aus. Bei der gleichen Temperatur und einer Konzentration von 60 % erhält man bereits über 75 % der Gesamtmenge als kristallines Trihydrat. Die obere Konzentrationsgrenze beträgt 73,65 %, da der Wassergehalt des Trihydrates selbst 26,35 % beträgt. So erhalt man bei der Kristallisation von Lösungen, die mehr als 70 % Natrium-L- oder -D-pyroglutamat enthalten, das jeweilige Trihydrat praktisch quantitativ.

Bei Erniedrigung der Temperatur der Lösung genügen bereits niedrigere Konzentrationen, um eine Kristallisation zu bewirken. So kann man bei 0 °C aus einer 48 %-igen Lösung kristallines Natrium-L- oder -D-pyroglutamat-trihydrat erhalten. Bei -20 °C kristallisiert bereits eine 40 %-ige Lösung. Tiefere Temperaturen sind nicht mehr empfehlenswert, da zusätzlich das Wasser gefriert. Für die obere Temperaturgrenze ist das Limit 42 °C, da dies der Schmelzpunkt von kristallinem Natrium-L- oder -D-pyroglutamat-trihydrat ist.

Bei der Kristallisation einer Lösung, die ein Gemisch aus Natrium-L- und -D-pyroglutamat enthält, kristallisiert jeweils nur das Enantiomere, das in höherer Konzentration vorliegt. Beträgt der Anteil des in geringerer Konzentration vorliegenden Enantiomeren jedoch mehr als 15 Molprozent, so findet keine Kristallisation des anderen Enantiomeren mehr statt.

Das erfindungsgemäße Verfahren kann deshalb auch dazu genutzt werden, um aus Mischungen von Natrium-L- und -D-pyroglutamat, die eines der beiden Enantiomeren zu mehr als 85 Molprozent enthalten, dieses in optisch reiner Form abzutrennen.

Bei dem erfindungsgemäßen Verfahren spielt es im Grunde keine Rolle, wie man die wäßrigen Lösungen des Natrium-L- oder -D-pyroglutamats herstellt. Eine Möglichkeit besteht darin, L- oder -D-Pyroglutaminsäure in wäßriger Lösung mit Natronlauge zu neutralisieren. Eine andere Möglichkeit ist die Auflösung von festem Natrium-L- oder -D-pyroglutamat, das man durch Schmelzen von Natrium-L- oder -D-glutamat-monohydrat erhalten kann, in Wasser.

Die Erfindung wird durch die nachfolgenden Beispiele naher erläuert:

Beispiel 1:

In 200 ml Wasser wurden 129,1g (1 Mol) L-Pyroglutaminsäure suspendiert. Unter Wasserkühlung wurden 250 ml (1 Mol) 4N Natronlauge zugetropft. Dabei ging die L-Pyroglutaminsäure als Natriumsalz vollständig in Lösung. Der pH-Wert betrug 8,3. Am Rotationsverdampfer wurden bei einer Badtemperatur von 45 °C und einem Druck von 25 mbar 320 ml Wasser abdestilliert. Der Wassergehalt der verbleibenden Lösung betrug 39 % (Titration nach Karl-Fischer). Beim Abkühlen auf 20 °C fielen 158 g (77 % der Theorie) farbloses Natrium-L-2-Pyrrolidon-5-carboxylat-trihydrat als grobkristalliner Niederschlag vom Schmelzpunkt 42 °C aus.

$C_5H_6 NNaO_3.3 H_2O$ (205,14)

Berechnet: C 29,26 % H 5,85 % N 6,82 % $H_2O$ 26.35 %

Gefunden: C 29,44 % H 5,98 % N 6,76 % $H_2O$ 26,44 %

$[\alpha]_D^{20}$ : - 18,68 ° (c = 4, $H_2O$),

$^1$H-NMR ($d^6$-DMSO) :$\delta$ = 1,80 - 2,15 (m; 4H, $CH_2$-$CH_2$), 3,51 (s; 6H, 3 $H_2O$), 3,69 (t; 1H, CH), 7,66 (s; 1H, NH).

IR (KBr) : 3700 - 2800 (breit, s), 1678 (s), 1593 (s), 1412 (m), 1300 (m), 1155 (w), 1104 (w), 1043 (w), 1009 (w), 723 (w) $cm^{-1}$.

Beispiel 2:

Analog Beispiel 1 wurde eine 50 %-ige Lösung von Natrium-L-pyroglutamat in Wasser hergestellt. Bei 20 °C bildete sich kein kristalliner Niederschlag. Beim Abkühlen auf 0 °C wurden 52 % der Theorie kristallines Natrium-L-pyroglutamat-trihydrat erhalten.

Beispiel 3:

Analog Beispiel 1 wurde eine 40 %-ige Lösung von Natrium-L-pyroglutamat in Wasser hergestellt. Beim Abkühlen auf -20 °C wurden 31 % der Theorie kristallines Natrium-L-pyroglutamat-trihydrat erhalten.

Beispiel 4:

Analog Beispiel 1 wurde eine 60 %-ige Lösung von Natrium-D-pyroglutamat in Wasser hergestellt. Als Ausgangsprodukt wurde D-Pyroglutaminsäure eingesetzt. Beim Abkühlen auf 20 °C wurden 74 % der Theorie farbloses, kristallines Natrium-D-2-pyrrolidon-5-carboxylat-trihydrat vom Schmelzpunkt 42 °C erhalten.

$[\alpha]_D^{20}$ : + 18,74 ° (c = 4, $H_2O$).

Beispiel 5:

100 g (0,66 Mol) Natrium-L-pyroglutamat, das durch Schmelzen von Natrium-L-glutamat-monohydrat erhalten worden war, wurden bei 50 °C in 43 ml Wasser gelöst. Die 70 %-ige Lösung wurde auf 20 °C abgekühlt. Dabei fielen 130 g (96 % der Theorie) Natrium-L-pyroglutamat-trihydrat als farblose Kristalle aus.

Beispiel 6:

In 20 ml Wasser wurden 11,62 g (0,09 Mol) L-Pyroglutaminsäure und 1,29 g (0,01 Mol) D-Pyroglutaminsäure suspendiert. Unter Wasserkühlung wurden 25 ml (0,1 Mol) 4N Natronlauge zugetropft. Am Rotationsverdampfer wurden 32 ml Wasser abdestilliert. Der Rückstand wurde auf 20 °C abgekühlt. Dabei fielen 12,3 g (67 % der Theorie) farbloses Natrium-L-pyroglutamat-trihydrat vom Schmelzpunkt 42 °C aus.

$[\alpha]_D^{20}$ : - 18,55 ° (c = 4, $H_2O$).

Zur exakten Bestimmung der Enantiomerenreinheit wurde eine Probe nach Derivatisierung mit zunächst Methanol/Chlorwasserstoff und anschließend Trifluoressigsäureanhydrid mittels einer chiralen GC-Säule analysiert (Chirasil-Val; 1 = 10 m; Lösungsmittel: Dichlormethan/Diethylketon (1 : 1); T = 110 °C). Nach Vergleich mit den Referenzsubstanzen L-Pyroglutaminsäure und D-Pyroglutaminsäure bestand die Probe aus 99,8 % L- und 0,2 % D-Enantiomer.

Beispiel 7:

Analog Beispiel 6 wurde eine Mischung aus 85 Molprozent Natrium-L-pyroglutamat und 15 Molprozent Natrium-D-Pyroglutamat kristallisiert. Bei einer Konzentration von 60 % in der Lösung wurden beim Abkühlen auf - 10 °C 12 % der Theorie Natrium-L-pyroglutamat-trihydrat erhalten.
$[\alpha]_D^{20}$ : - 18,3 ° (c = 4, $H_2O$).

## Patentansprüche

1. Natrium-L-2-pyrrolidon-5-carboxylat-trihydrat.

2. Natrium-D-2-pyrrolidon-5-carboxylat-trihydrat.

3. Verfahren zur Gewinnung von Natrium-L- oder -D-2-pyrrolidon-5-carboxylat-trihydrat gemäß Anspruch 1 oder 2, dadurch gekennzeichnet, daß man in einer wäßrigen Lösung von Natrium-L- oder -D-2-pyrrolidon-5-carboxylat, die höchstens 15 Molprozent des jeweiligen anderen Enantiomeren enthält, die Konzentration in dem Bereich zwischen 40 und 73,65 Gewichtsprozent und die Temperatur in dem Bereich zwischen -20 °C und +42 °C so aufeinander abstimmt, daß die Löslichkeit des jeweiligen Trihydrats überschritten wird.

## Claims

1. Sodium L-2-pyrrolidone-5-carboxylate trihydrate.

2. Sodium D-2-pyrrolidone-5-carboxylate trihydrate.

3. A process for isolating sodium-L- or -D-2-pyrrolidone-5-carboxylate trihydrate according to Claim 1 or 2, characterized in that in an aqueous solution of sodium-L- or -D-2-pyrrolidone-5-carboxylate that contains no more than 15 mole % of the other enantiomer concerned the concentration in the range between 40 and 73.65 % by weight and the temperature in the range between -20 °C and +42 °C are so matched to each other that the solubility of the trihydrate concerned is exceeded.

## Revendications

1. Carboxylate-5-trihydraté-pyrrolidone-2 de sodium L.

2. Carboxylate-5-trihydraté-pyrrolidone-2 de sodium D.

3. Procédé pour obtenir du carboxylate-5-trihydraté-pyrrolidone-2 de sodium L ou D selon la revendication 1 ou la revendication 2, caractérisé en ce que - dans une solution aqueuse de carboxylate-5-pyrrolidone-2- de sodium L ou D, qui contient au maximum 15 pour cent en mol de l'autre énantiomère respectif, on ajuste l'une avec l'autre la concentration dans la zone entre 40 et 73,65 pour cent en poids et la température dans la zone entre -20°C et + 42°C de façon, que la solubilité du trihydrate correspondant soit dépassée.